(19) **Europäisches Patentamt**

**European Patent Office**

**Office européen des brevets**

(11) **EP 0 660 638 B1**

(12) **EUROPEAN PATENT SPECIFICATION**

(45) Date of publication and mention
of the grant of the patent:
**22.01.2003 Bulletin 2003/04**

(51) Int Cl.⁷: **H04R 1/02**, H04R 1/28,
H04N 5/60, F24F 3/16,
A61N 1/44

(21) Application number: **94400255.9**

(22) Date of filing: **07.02.1994**

(54) **Speaker system with an anion generator and a television set using the speaker system**

Lautsprechersystem mit Anionengenerator und Fensehgerät mit diesem Lautsprechersystem

Système de haut-parleur avec générateur d'anions et poste de télévision d'un tel système

(84) Designated Contracting States:
**DE FR GB**

(30) Priority: **21.12.1993 KR 9328796**
**28.01.1994 KR 941568**

(43) Date of publication of application:
**28.06.1995 Bulletin 1995/26**

(73) Proprietor: **LG ELECTRONICS INC.**
**Seoul (KR)**

(72) Inventors:
• **Kim, Chan Han**
**Daeku (KR)**

• **Lee, Deog Jin**
**Kumi, Kyungsanbook-Do (KR)**
• **Hwang, Kyong Soo**
**Daeku (KR)**

(74) Representative: **Fort, Jacques**
**CABINET PLASSERAUD**
**84, rue d'Amsterdam**
**75440 Paris Cedex 09 (FR)**

(56) References cited:
**DE-A- 2 452 824**         **DE-A- 2 453 235**
**DE-A- 2 509 767**         **FR-A- 2 423 944**
**FR-A- 2 559 981**         **US-A- 3 840 788**

## Description

The present invention relates in general to a speaker system with an anion generator and a television set using the speaker system, and more particularly to a speaker system and a television set for generating anions and spreading effectively said generated anions by means of the sound pressure of the speaker.

Conventionally, when watching a program from a television set or a video cassette tape recorder or listening to music from an audio which are usually provided in an area where many people are crowded or some persons are gathered, the watchers or listeners could not be free from various harmful elements such as dust, air polluted by smoking, etc-.

It has already been proposed (DE-A- 2452824) to provide a radio receiver and an air ionisation generator in a common box and to energize them from a common supply. From this document it is moreover known to place the discharge needle of the anion generator in front of the cone of a speaker. It has also been proposed to provide a box accomodating a speaker and an amplifier with a lower inlet port and an upper outlet port for air to flow through the box (FR-A-2423944).

It is an object of the present invention to provide a speaker system with an anion generating function and a television set using the speaker system in which anions are generated to offer people a convenient environment when watching a television or video cassette tape recorder program or enjoying music from an audio system free from the above mentioned harmful problems. It is a more specific object to provide a television set and a speaker system generating anions for people to live in a better environment. The anions are obtained by an anion generator provided in the speaker system and widely spread by the sound pressure of the speaker.

To achieve the above mentioned object, there is provided a speaker system according to claim 1. Other features are defined in the dependant claims.

The above and other objects, features and advantages of the invention will be more clearly understood from the following description taken in conjunction with the accompanying drawings, wherein:

Fig. 1 is a sectional view illustrating a first embodiment of a speaker system with an anion generator of the present Invention

Fig. 2 is a sectional view illustrating a speaker system with an anion genenator not falling under the present invention but given for illustration;

Fig. 3 is a sectional view illustrating a second embodiment of the present invention;

Figs. 4A and 4B are schematic views illustrating a television cabinet with a speaker box therein;

Figs. 5 and 6 are sectional views illustrating a part of a speaker of a television set provided with the anion generator therein, according to the first and second embodiment respectively;

Fig. 7 is a sectional view illustrating the structure of another embodiment of the television set with the anion generator therein;

Fig. 8 is a sectional view illustrating the structure of another embodiment of the television set with the anion generator therein.

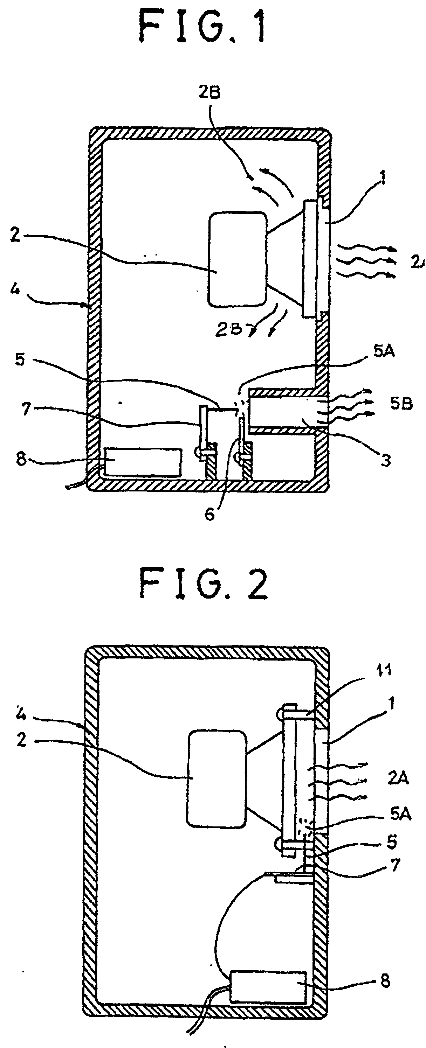

Referring to Fig. 1, there is illustrated a sectional view illustrating the first embodiment of a speaker system with an anion generator according to the present invention. As illustrated in this drawing, the speaker system with the anion generator includes a speaker box 4 having a sound outputting hole 1 for outputting a front output sound 2A to the forward direction of a speaker 2 and a port 3, provided in the front face of the speaker box and below the sound outputting hole 1, for outputting a rear output sound 2B output to the backward direction of the speaker 2, provided at an inner position of the speaker box 4, for generating the front output sound 2A to the forward direction and the rear output sound 2B to the backward direction due to the vibrating action of the speaker 2, a discharge needle 5 for generating anions provided at a close position to the port 3 of the speaker box 4, a facing electrode 6 for helping a discharging process of the discharge needle 5 to generate anions, a printed circuit board 7 on which the discharge needle is fixed, and a high voltage generator 8 for applying high voltage to the discharge needle 5 through the circuit board 7.

The anions generating and spreading process according to the first embodiment of the present invention will be provided as follows:

When turning on an audio system or a VTR, the speaker 2 vibrates in response to the sound signal; as a result of the vibration of the speaker 2, the front sound output 2A is provided through the sound outputting hole 1 outputting to the forward direction, and the rear sound output 2B is provided to the port 3 through the inside space of the speaker box 4.

When electric power is applied to the high voltage generator 8, the high voltage generator 8 applies the produced high voltage to the discharge needle 5 fixed on the circuit board 7. Discharge occurs from the discharge needle 5 with respect to the facing electrode 6 and generates anions (5A) as a result of this process.

The anions generated by this process are spread by the pressure of the rear sound output 2B of the speaker 2 through the port 3, into the indoor space in which this system is placed.

This process operates by means of the sound pressure of the rear sound output 2B generated by the vibration of the speaker 2, and so the indoor space in which the speaker system is placed will be provided with anions. The facing electrode 6 helps the discharging process of the discharge needle 5 to generate anions 5A.

Fig. 2 is a sectional view of a speaker system with an anion generator not falling under the present invention but given for illustration.

Referring to Fig. 2, the system comprises the speaker box 4 having a sound outputting hole 1 for outputting the front sound output 2A to the forward direction of the speaker 2. The speaker 2 located inside the speaker box 4 provides the front face sound output 2A output to the forward direction in addition to sound in the backward direction. The discharge needle 5 generates anions at the position in which the front sound output 2A of the speaker box 4 occurs, that is to say between the sound outputting hole 1 and the front face of the speaker 2. The discharge needle 5 is fixed on the circuit board 7. The high voltage generator 8 applies high voltage to the discharge needle 5 through the circuit board 7. In addition, the discharge needle 5 passes through the holder 11 provided between the sound outputting hole 1 and the front face of the speaker 2.

In this system, there is no facing electrode, but if many anions are needed, such an electrode may be provided.

The spreading process of the anions according to the system of Fig. 2 will now be described.

When turning on an audio or VTR machines, the speaker 2 vibrates in response to the sound, as a result of the vibration, the front sound output 2A is provided through the sound outputting hole 1 to the forward direction and the rear sound output 2B is provided to the port 3 through the inside space of the speaker box 4.

When electric power is applied to the high voltage generator 8, the high voltage generator 8 applies high voltage to the discharge needle 5 fixed on the circuit board 7.

The anions generated by this process are spread by the sound pressure of the front sound output 2A of the speaker 2 through the sound outputting hole 1 are into the indoor space in which this system is located.

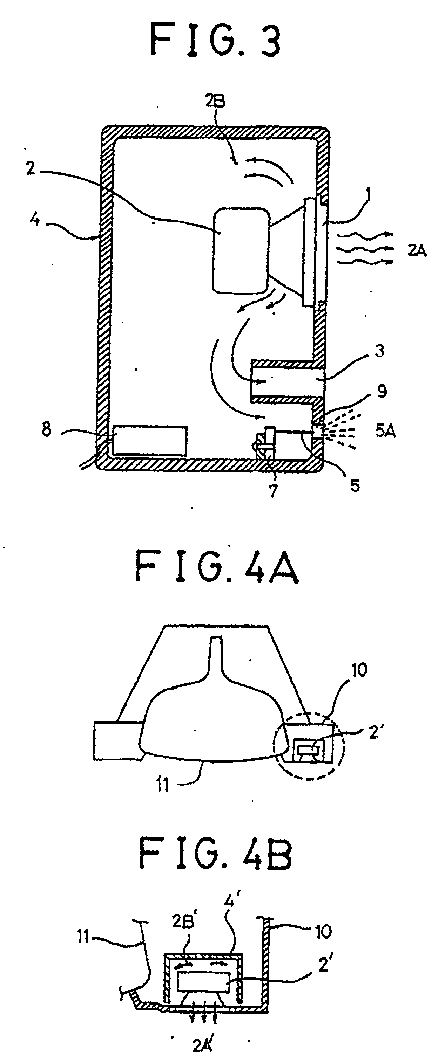

Referring to Fig. 3, the second embodiment of the present invention comprises the speaker box 4 including the sound outputting hole 1 outputting the front sound output 2A to the forward direction of the speaker 2, and the port 3, provided below the sound outputting hole 1, for outputting sound output 2B rearwardly produced by the speaker 2. The speaker 2, inside the speaker box 4 provides the front sound output 2A forwardly and the rear sound output 2B backwardly. The anions discharging port 9 is provided at a predetermined position of the speaker box. The discharge needle 5 is provided near to or inside the anions discharging hole 9 of the speaker box 4 for generating anions. The circuit board 7 supports the discharge needle 5. The high voltage generator 8 applyies high voltage to the discharge needle 5 through the circuit board 7.

When turning on an audio or VTR machine, the speaker 2 vibrates in response to the audio input. As a result of the vibration, the front sound output 2A is provided through the sound outputting hole 1 forwardly and the rear sound output 2B is provided to the port 3 through the inside space of the speaker box 4.

When electric power is applied to the high voltage generator 8, the high voltage generator 8 applies high voltage to the discharge needle 5 fixed at the circuit board 7.

The anions generated by this process are spread by the sound pressure of the speaker 2 into the indoor space in which this system is located.

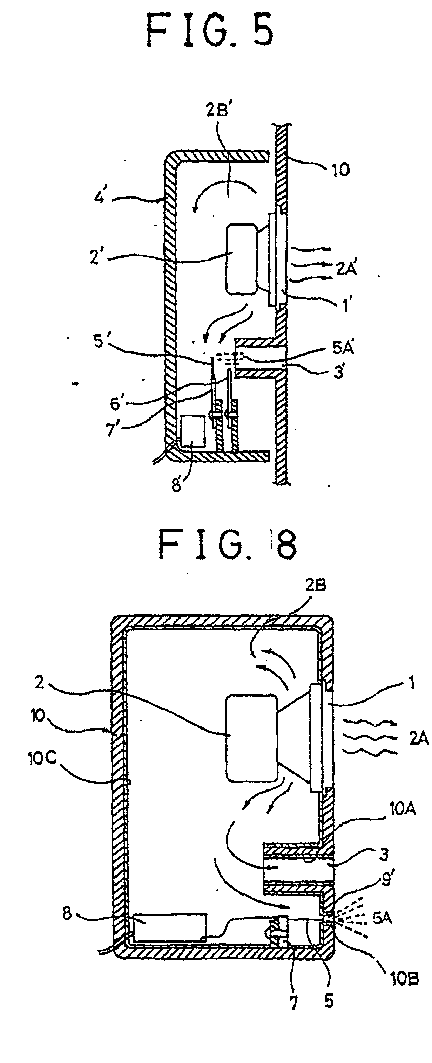

Figs. 4A, 4B and 5 illustrate a speaker 2' provided in the speaker cabinet 10 of a television set, with the anions generating means in the speaker box 4' according to the first embodiment of the present invention.

Thus, when electric power is applied to the television set, sound is outputted from speaker 2', and anions(5A') of the discharge needle 5' are spread in the indoor space by the sound pressure of the rear sound 2B' when front and rear sound 2A' and 2B' are produced in response to the sound signal received by the speaker 2'.

In the drawings, reference numeral 11 denotes a CRT (cathode ray tube), and the discharge needle 5' may be provided at a predetermined position inside the cabinet 10, assuming the entire portion of the television cabinet 10 is the speaker box.

In Figs. 4-5, reference numerals corresponding to those of the preceding figures are used with a prime mark.

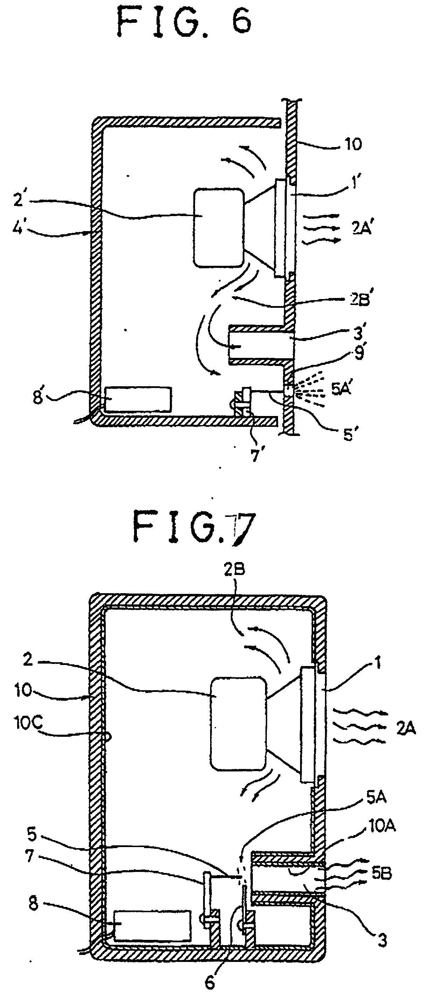

**Fig. 6** illustrates a television set with an anion generator according to the second embodiment, and it may be adapted in cases that the anion generator is provided in the speaker of the television box and the anions 5A' are discharged (evacuated) by the sound pressure of the rear sound output 2B' by placing the discharge needle 5' in the sound outputting hole 9'.

**Fig. 7** is a sectional view illustrating a part of the speaker of a third embodiment of the television set with an anion generator. As illustrated in Fig. 7, the television set with the anion generator according to the present invention comprises a sound outputting hole 1 for outputting front sound 2A forwardiy of the speaker 2 located inside the cabinet; the speaker also generates rear output sound 2B backwardly of the speaker 2. The discharge needle 5 for generating anions is near the port 3 of the cabinet 10 and fixed on the circuit board 7. The cabinet accomodates high voltage generator 8 for applying the high voltage to the discharge needle 5 through the circuit board 7. Electromagnetic preventives 10C (antistatic agents) are coated on the inside wall of the cabinet 10 for preventing an electron sheath from forming on the inside wall of the cabinet 10.

The anion generating and spreading process will now be described.

When turning on an audio or VTR machine, the speaker 2 vibrates. As a result of the vibration, the front sound output 2A is provided through the sound outputting hole 1 forwardly and rear sound output 2B is provided to the port 3 through the inside space of the speaker box 4.

When electric power is applied to the high voltage generator 8, the discharge needle 5 fixed on the circuit board 7 receives high voltage and there is a discharge to the facing electrode 6, so that anions 5A are generated according to the formula:

$$e^- + O_2 \rightarrow O_2^-$$

The generated anions 5A ($O_2^-$) are discharged (evacuated) through the port 3 by the sound pressure of the rear sound output 2B of the speaker 2 and the air receiving the rear sound output 2B with said anions is widely spread in the indoor space.

The generated anions are widely spread by the sound pressure of the speaker, although the electrons ($e^-$) generated along with anions reach the inner surface of the cabinet 4 faster than the ion $O_2^-$ since the electrons ($e^-$) move faster than the ions $O_2^-$, between the cabinet 10, nonconductor, and the inner space. Adhesion of the electrons ($e^-$) on the inner surface of the cabinet 10 may occur.

It may be possible to coat electromagnetic preventives (10C) on the inner surface of the anions discharging port 3. Then, since electromagnetic preventives (10C) are also coated on the surface of the anions discharging port 3, the formation of an electron sheath 9 (not illustrated) is eliminated, thus the anions generated as described above can be spread into the indoor space while freely passing through the port 3 providing enough space for the generated anions.

U.V (ultraviolet) protection material may be provided on the inner surface of the cabinet 10.

In another embodiment the electromagnetic preventives are within the plastic materials of the cabinet. In this case the adhering phenomenon of the electrons $e^-$ occurring during anions generation onto the cabinet wall is avoided.

In another embodiment paper sheets or PVC sheets coated with U.V. materials could be provided. Adhesion of the electrons $e^-$ occuring during anions generation onto the cabinet wall including electromagnetic preventives is avoided.

In another embodiment the electromagnetic preventives (10C) are coated on the inner surface of the cabinet 4 and U.V. coating tape is bonded on the port 3. Alternatively it is possible to coat the electromagnetic preventives (10C) on the port 3 and to provide U.V. coating tape on the inner surface of the cabinet 10.

Fig. 8 illustrates another embodiment. The embodiment of Fig. 8 comprises a sound outputting hole 1 for outputting the front sound 2A forwardly of the speaker 2. A port 3 is provided for outputting the rear sound output 2B generated backwardly of the speaker 2 located inside the cabinet 10. A discharge needle 5 for generating the anions is located near the anions discharging hole 5A or inside the anions discharging hole 3A provided at a predetermined position of the cabinet wall. The cabinet accomodates the circuit board 7 on which the discharge needle 5 is fixed. The high voltage generator 8 applies high voltage to the discharge needle 5 through the circuit board 5. Electromagnetic preventives (10C) prevent the formation of an electron sheath on the inner portion of the cabinet 10. U:V. materials (10A) preventing the formation of an electron sheath are attached on the inner wall of the sound outputting port 3.

As the anion generating and spreading process according to that embodiment of the present invention is the same as that of the third embodiment, no further description is given.

Although preferred embodiments of the present invention have been disclosed for illustrative purposes, those skilled in the art will appreciate that various modifications, additions and substitutions are possible within the scope defined by the claims.

## Claims

1. A speaker system with an anion generator, comprising:

   a speaker (2) and anion generating means (5,6) located in a speaker box (4; 10) which has a sound output hole (1) in a front face thereof for outputting forwardly generated sound from the speaker (2) in the forward direction,

   **characterized in that** a port (3) is provided in the front face of said speaker box (4; 10) below said sound output hole (1) for outputting sound rearwardly generated by the speaker (2) within the speaker box (4; 10) to the forward direction, **in that** said anion generating means include a discharge needle (5) being located nearby the interior opening of said port (3) or adjacent or inside an anions discharging hole (9,9') provided in the front face of said speaker box (4;4';10) and below said port (3), such that the sound pressure of the rearwardly generated sound from the speaker (2) spreads the anions generated from said discharge needle (5) of the anion generating means (5,6) respectively through said port (3) or through said anions discharging hole (9,9') into the indoor space in which speaker system is located.

2. The speaker system of claim 1, wherein said anions generating means (5,6) further includes a high voltage generator (8) a circuit board (7) for applying the high voltage generated by said high voltage generator (8) to said discharge needle (5), and an electrode (6) facing said discharge needle (5) for enabling the discharge needle (5) to produce anions.

3. The speaker system of claim 2, wherein said high voltage generator (8) is located at one side of the bottom of said speaker box (4; 10).

4. A television set comprising a speaker system according to any one of the preceding claims, **characterized in that** said speaker system has a cabinet constituting said speaker box (10).

5. A television set according to claim 4, **characterized in that** said cabinet comprises an antistatic agent for preventing an electron sheath generated by the anions within said cabinet.

6. A television set according to claim 5, **characterized in that** said port (3) or said anions discharging hole (9') is coated with said antistatic agent (10B;10C).

7. A television set according to claim 4, **characterized in that** said cabinet comprises an ultraviolet material for preventing an electron sheath generated by the anions within said cabinet.

8. A television set according to claim 7, **characterized in that** said ultraviolet material is coated on the inside wall surface of said cabinet.

9. A television set according to claim 7, **characterized in that** said ultraviolet material is coated in the form of a tape.

10. A television set according to claim 7, **characterized in that** an antistatic agent is coated on the inside wall of said port or said anions discharging hole.


**Patentansprüche**

1. Lautsprechersystem mit einem Anionenerzeuger, umfassend:

einen Lautsprecher (2) und eine anionenerzeugende Einrichtung (5, 6), die in einem Lautsprechergehäuse (4; 10) gelegen sind, das eine Tonausgabeöffnung (1) in einer vorderen Fläche davon zum nach vorne Ausgeben von Ton, der durch den Lautsprecher (2) in der Vorwärtsrichtung erzeugt ist, besitzt,

**dadurch gekennzeichnet, dass** ein Durchgang (3) in der vorderen Fläche des Lautsprechergehäuses (4; 10) unterhalb des Ausgabelochs (1) zum Ausgeben von Ton, der rückwärtig durch den Lautsprecher (2) innerhalb des Lautsprechergehäuses (4; 10) erzeugt wird, nach vorne, vorgesehen ist, dass die anionenerzeugende Einrichtung eine Entladungsnadel (5) einschließt, die nahe der inneren Öffnung des Durchgangs (3) oder benachbart oder innerhalb eines anionenausgebenden Lochs (9, 9') gelegen ist, das in der vorderen Fläche des Lautsprechergehäuses (4; 4'; 10) und unterhalb des Durchgangs (3) gelegen ist, so dass der Schalldruck des rückwärtig erzeugten Tons von dem Lautsprecher (2) die Anionen, die durch die Entladungsnadel (5) der anionenerzeugenden Einrichtung (5, 6) erzeugt werden, jeweils durch den Durchgang (3) oder das Anionenausgabeloch (9, 9') in den Innenraum, in welchem das Lautsprechersystem gelegen ist, ausbreitet.

2. Lautsprechersystem nach Anspruch 1, worin die anionenerzeugende Einrichtung (5, 6) weiter einen Hochspannungsgenerator (8), eine Leiterplatte (7) zum Aufbringen der durch den Hochspannungsgenerator (8) erzeugten Hochspannung auf die Entladungsnadel (5) und eine Elektrode (6), die der Entladungsnadel (5) zugewandt ist, um es der Entladungsnadel (5) zu ermöglichen, Anionen zu erzeugen, einschließt.

3. Lautsprechersystem nach Anspruch 2, worin der Hochspannungsgenerator (8) auf einer Seite des Bodens des Lautsprechergehäuses (4; 10) gelegen ist.

4. Fernseheinrichtung, umfassend ein Lautsprechersystem nach einem der vorhergehenden Ansprüche, **dadurch gekennzeichnet, dass** das Lautsprechersystem einen Abschnitt besitzt, der das Lautsprechergehäuse (10) bildet.

5. Fernseheinrichtung nach Anspruch 4, **dadurch gekennzeichnet, dass** der Abschnitt ein antistatisches Mittel zum Verhindern eines Elektronenschirms, der durch die Anionen innerhalb des Abschnitts erzeugt ist, aufweist.

6. Fernseheinrichtung nach Anspruch 5, **dadurch gekennzeichnet, dass** der Durchgang (3) oder das Anionenausgabeloch (9) mit dem antistatischen Mittel (10B; 10C) beschichtet ist.

7. Fernseheinrichtung nach Anspruch 4, **dadurch gekennzeichnet, dass** der Abschnitt ein ultraviolettes Material zum Verhindern eines Elektronenschildes, der durch die Anionen innerhalb des Abschnitts erzeugt wird, aufweist.

8. Fernseheinrichtung nach Anspruch 7, **dadurch gekennzeichnet, dass** das ultraviolette Material auf der inneren Wandfläche des Abschnitts schichtartig aufgetragen ist.

9. Fernseheinrichtung nach Anspruch 7, **dadurch gekennzeichnet, dass** das ultraviolette Material in der Form eines Bandes schichtartig aufgebracht ist.

10. Fernseheinrichtung nach Anspruch 7, **dadurch ge-**

**kennzeichnet, dass** ein antistatisches Mittel auf der Innenwand des Durchgangs oder der Anionenausgabeöffnung schichtartig aufgetragen ist.

## Revendications

1. Système de haut-parleur comportant un générateur d'anions comprenant :

   un haut-parleur (2) et des moyens (5,6) de production d'anions, situés dans un boîtier (4;10) du haut-parleur, qui possède un trou (1) de sortie du son formé dans une face avant de ce boîtier de manière à délivrer un son produit vers l'avant à partir du haut-parleur (2) en direction de l'avant,

   **caractérisé en ce qu'**un orifice (3) est prévu dans la face avant dudit boîtier (4;10) de haut-parleur au-dessous dudit trou (1) de sortie du son pour la délivrance du son produit vers l'arrière par le haut-parleur (2) à l'intérieur du boîtier (4;10) du haut-parleur, dans la direction tournée vers l'avant, **en ce que** lesdits moyens de production d'anions comprennent une aiguille de décharge (5) disposée à proximité de l'ouverture intérieure dudit orifice (3) ou au voisinage ou à l'intérieur d'un trou (9,9') de décharge d'anions prévu dans la face avant dudit boîtier (4;4';10) du haut-parleur et au-dessous dudit orifice (11), de telle sorte que la pression acoustique du son produit vers l'arrière par le haut-parleur (2) étale les anions produits par ladite aiguille de décharge (5) desdits moyens (5,6) de production d'anions respectivement par ledit orifice (3) ou par ledit trou (9,9') de décharge d'anions, dans l'espace intérieur dans lequel le système de haut-parleur est situé.

2. Système de haut-parleur selon la revendication 1, dans lequel lesdits moyens (5,6) de production d'anions incluent en outre un générateur de haute tension (8), une carte de circuits (7) pour appliquer la haute tension produite par ledit générateur de haute tension (8) à ladite aiguille de décharge (5), et une électrode (6) tournée vers ladite aiguille de décharge (5) pour permettre à ladite aiguille de décharge (5) de produire des anions.

3. Système de haut-parleur selon la revendication 2, dans lequel ledit générateur de haute tension (8) est situé sur un côté du fond dudit boîtier (4;10) du haut-parleur.

4. Appareil de télévision comprenant un système de haut-parleur selon l'une quelconque des revendications précédentes, **caractérisé en ce que** ledit système de haut-parleur comporte un coffret constituant ledit boîtier (10) du haut-parleur.

5. Appareil de télévision selon la revendication 4, **caractérisé en ce que** ledit coffret comprend un agent antistatique destiné à empêcher une couche d'électrons produite par les anions à l'intérieur dudit coffret.

6. Appareil de télévision selon la revendication 5, **caractérisé en ce que** ledit orifice (3) ou ledit trou (9') de décharge des anions est recouvert par ledit agent antistatique (10B;10C).

7. Appareil de télévision selon la revendication 4, **caractérisé en ce que** ledit coffret comprend un matériau résistant aux ultraviolets pour empêcher une couche d'électrons produite par les anions à l'intérieur dudit coffret.

8. Appareil de télévision selon la revendication 7, **caractérisé en ce que** ledit matériau de protection contre les ultraviolets est déposé sur la surface de paroi intérieure dudit coffret.

9. Appareil de télévision selon la revendication 7, **caractérisé en ce que** ledit matériau de protection contre les ultraviolets est déposé sous la forme d'une bande.

10. Appareil de télévision selon la revendication 7, **caractérisé en ce qu'**un agent antistatique est déposé sur la paroi intérieure dudit orifice ou dudit trou d'évacuation des anions.

# FIG. 1

# FIG. 2

# FIG. 3

# FIG. 4A

# FIG. 4B

8

# FIG. 5

# FIG. 8

# FIG. 6

# FIG. 7